# EUROPEAN PATENT SPECIFICATION

(11) **EP 0 576 674 B1**
(45) Date of publication and mention of the grant of the patent: **09.06.1999**
(21) Application number: 92905782.6
(22) Date of filing: 06.03.1992
(51) Int. Cl.: C12N 15/74, C12N 1/13

(54) **NOVEL PLASMID**
NEUARTIGES PLASMID
NOUVEAU PLASMIDE

(30) Priority: 08.03.1991 JP 6777491
(43) Date of publication of application: 05.01.1994
(73) Proprietor: HAGIWARA, Yoshihide, Takarazuka-shi, Hyogo 665 (JP)
(72) Inventor: HAGIWARA, Hideaki, Takarazuka-shi, Hyogo 665 (JP); TAKESHIMA, Yasunobu, Kasai-shi, Hyogo 679-01 (JP)
(74) Representative: Cresswell, Thomas Anthony
(86) International application number: JP9200267
(87) International publication number: WO9215692

(56) References cited:
- EP-A- 0 533 942
- JP-A- 62 175 192
- Gene, Vol. 19, 1982, K. SHINOZAKI et al., "Cloning and characterization of a plasmid DNA from Anacystis-nidulans 6301", p. 221-224.

## Description

### Technical Field

This invention relates to a novel plasmid, and relates more detailedly to a shuttle vector plasmid replicable in the cells of both Escherichia coli and cyanobacteria, which contains a DNA fragment containing the OriA region of plasmid pBA1 derived from Anacystis nidulans, a cyanobacterium.

### Background Art

A cyanobacterium Anacystis nidulans has a photosynthetic mechanism similar to those of higher plants, especially red algae, and can biosynthesize organic substances from water, carbon dioxide and slight inorganic salts and thus autotrophically proliferate. Further, A. nidulans is unicellular and makes colonies on an agar medium, and especially R2 strain tends to take DNAs into the cell and is thus possible to transform according to microbial and genetic methods.

Thus recently, many cloning vectors using endogenous plasmids in the A. nidulans R2 strain (for example, pUH24 and pUH25) have been developed [refer to C.A.M.J.J. von der Hondel et al. Proc. Natl. Acad. Sci. USA 77(3); 1570-1574 (1980); C. J. Kuhlemeier et al., Mol. Gen. Genet. 184:249-254 (1981); L. A. Sherman and P. van de Putte, J. Bacteriol. 150(1):410-413 (1982); S. Gendel et al., J. Bacteriol. 156(1):148-154 (1983); S. S. Golden and L. A. Sherman, J. Bacteriol. 155(3):966-972 (1983); C. J. Kuhlemeier et al., Plasmid 10:156-163 (1983); C. J. Kuhlemeier et al., Gene 31; 109-116 (1984); D. E. Laudenbach et al., Mol. Gen. Genet. 199:300-305 (1985); M. Y. Gruber et al., Curr. Microbiol. 15:265-268 (1987), etc.], and used for example for introduction of foreign genes and cloning of genes.

As examples of heterogenous proteins expressed using the strain R2 are known human carbonic anhydrase and the lac IQ repressor protein of Escherichia coli [G. D. Price and M. R. Badger, Plant Physiol. 91:505-513 (1989)], α-amylase of Bacillus amyloliquefaciens A50 [I. V. Elanskaya and I. B. Morzunoba, Mol. Genet. Microbiol. Virusol. 0(9):7-1 (1989)], an insecticidal protein of B. sphaericus 1593M [N. Tandeau de Marsac et al., Mol. Gen. Genet. 209:396-398 (1987)], β-galactosidase of Escherichia coli [D. J. Scanlan et al., Gene. 90:43-49 (1990), M. R. Schaefer and S. S. Golden, J. Bacteriol. 171(7):3973-3981 (1989)1,, Mn-superoxide dismutase of Escherichia coli [M. Y. Gruber et al., Proc. Natl. Acad. Sci. USA 87:2608-2612 the (cI^{ts} repressor protein of λ phage [D. Friedberg and J. Seiiffers, Mol. Gen. Genet. 203:505-510 (1986)], disaturase of a cyanobacterium Synechosystis PCC 6803 strain (des A) [H. Wada et al., Nature 347:200-203 (1990], etc.

On the other hand, A. nidulans 6301 have been developed using its endogenous plasmid (pBA1) vectors (shuttle vectors with Escherichia coli) such as pBAS 18 [12 kb, K. Shinozaki et al., Gene. 19:221-225 (1982)] and pBAS 5 [14 kb, Kazuo Shinozaki, "Shokubutsu Idenshi Sosa Gijutsu" (Plant Gene Manipulation Techniques), supervised by Hikoyuki Yamaguchi, pages 98 to 110, published by CMC]. However, these vectors present difficult problems in gene manipulation, for example the size of a gene capable of being inserted into any of the vectors is restricted because these vectors have a length of 10 kb or more and each vector has only a small number of restriction endonuclease recognition sites usable for cloning. Further, the strain 6301 has a disadvantage that it does not easily take a gene DNA into its cell, compared with the strain R2, and as a result its transformation frequency is lower than that of the strain R2. As a method of enhancing the transformation frequency of the strain 6301, H. Daniell, et al. [Proc. Natl. Acad. Sci. USA 83:2546-2550 (1986)] propose that cells of the strain 6301 are treated with EDTA-lysozyme to convert them to permeaplasts, whereby to make it easy to take DNAs thereinto. In fact, they succeeded in enhancing the transformation frequency of the strain 6301 using this method, but it has problems, for example, that the manipulation takes much time and is complicated and further the cells damaged by the EDTA-lysozyme treatment.

Thus, for the purposes of enhancement of the transformation frequency of A. nidulans 6301, construction of a plasmid easy to handle on gene manipulation, etc., the present inventors, first, intensely studied miniaturization of a plasmid, and succeeded in creating a plasmid capable of remarkably enhancing the transformation frequency of the strain 6301 by making as small as possible the parts other than the replication initiation region using an endogenous plasmid (pBA1) of the strain 6301 as a base and at the same time by combining the resulting plasmid with a DNA fragment which contains the replication initiation region of the Col. E1 plasmid of Escherichia coli (OriE. region) and wherein a gene defined by the rop region (this region is also called rom region but referred to as rop region in the present specification) is removed, and further included the multicloning region of pUC 18, to complete this invention.

### Disclosure of the Invention

Thus according to this invention is provided a shuttle vector plasmid replicable in the cells of Escherichia coli and cyanobacteria, which contains
(a) the OriA region of plasmid pBA1 derived from Anacystis nidulans,
(b) the multicloning region comprising a sequence having various restriction endonuclease recognition sites and
(c) the region of colicin E1 plasmid, which contains the OriE region thereof and wherein the gene defined by the rop region thereof is removed.

The plasmid of this invention is further detailedly described below.

"The OriA region of plasmid pBA1 derived from Anacystic nidulans" (hereinafter sometimes abbreviated as "OriA") forming part of the plasmid of the invention is a gene fragment containing the replication initiation region derived from plasmid pBA1. Plasmid pBA1 has a molecular weight of (5.04 ± 0.26) x 10⁶ (by electron microscope analysis) or 5.2 x 10⁶ (by agarose gel electrophoresis analysis) [K. Shinozaki et al., Gene., 19:221-224 (1982)] and was derived from two endogenous plasmids of Anacystic nidulans 6301 (Synechococcus PPC 6301, ATCC 27144, UTEX 625 strain) which belongs to the genus cloococcus, blue-green algae. The size of the OriA region can generally be in the range of 2.8 to 8.0 kb., preferably 2.8 to 4.4. kb. Specific examples thereof are an OriA region-containing DNA fragment having a size of about 4.4. kb obtained by cutting pBA1 with restriction endonucleases XhoI and BamHI, an OriA region-containing DNA fragment having a size of about 3.3 kb obtained by cutting pBA1 with PvuII and BamHI, etc.

The "multicloning region" (hereinafter sometimes abbreviated as "MC region") is a sequence (which is also called polyinker) having various restriction endonuclease recognition sites. An example thereof is Col El plasmid pUS used as an Escherichia coli cloning vector, e.g., a DNA fragment of 55 bp having restriction endonuclease recognition sites of EcoRI, SacI, KpnI, SmaI, XmaI, BamHI, XbaI, SalI, AccI, HincII, PstI, SphI and Hind III. Further, MC regions derived from pUS 12, pUC 13, pUC 19, etc., and a MC region derived from a phage vector M 13 of Escherichia coli are also available. Still further, chemically synthesized MC regions are also available.

Further, "a region derived from Col E1 plasmid which contains the OriE region and wherein the gene defined by the rop region is removed" (hereinafter sometimes abbreviated as "OriE") used in combination of the OriA and MC regions in the invention means the replication initiation region of a Col E1 plasmid which is used in Escherichia coli and whose copy number is 20 to 30 per cell body. A representative example of such OriE is an OriE-containing DNA fragment which can be cut out from plasmid pBR 322 with restriction endonuclease PvuII, AvaI, BamHI, Eco 47III, EcoRV, PstI, etc., and whose size is 1.5 to 4.1 kb, preferably 1.5 to 2.5 kb, and there can further be used OriE-containing DNA fragments derived from plasmids pUC 18, etc.

Further, these OriE-containing DNA fragments can contain drug resistance genes such as an ampicillin resistance gene (Amp^{r}) which can be a marker in cloning.

So long as a plasmid provided by this invention has DNA fragments containing respectively the afore-mentioned three essential gene regions, i.e., OriA, MC region and OriE, it can further contain DNA fragments carrying other genetic informations, for example a DNA fragment containing an ampicillin resistance gene, an antibiotic resistance marker and/or a DNA fragment containing a chloramphenicol resistance gene, an antibiotic resistance marker. Typical examples of such plasmids are two plasmids named "plasmid pBAX 18" and "plasmid pBAX 20" respectively by the present inventors which substantially comprise DNA fragments containing three gene regions of OriA, MC region and OriE respectively and whose molecular weights are about 4.48 MDa (about 6.9 kb) and about 3.76 megadaltons (about 5.8 kb) respectively.

In the present specification, the molecular weight of plasmids is a value measured by the agarose gel electropheresis method.

These plasmids pBAX 18 and pBAX 20 are more detailedly described below.

### Brief Description of the Drawings

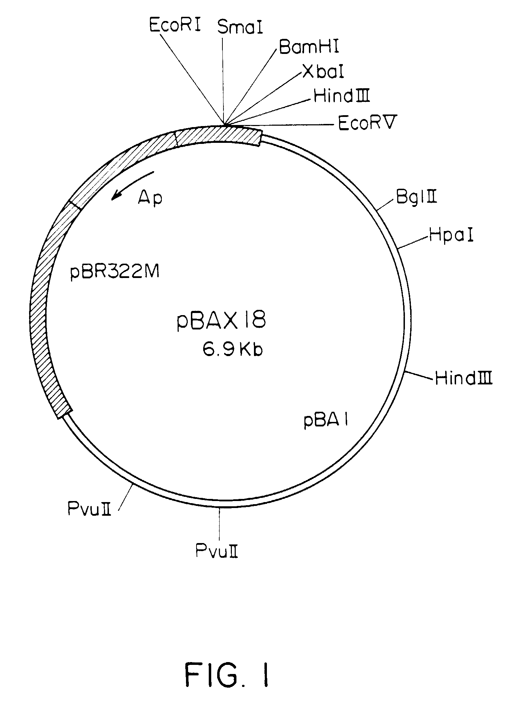

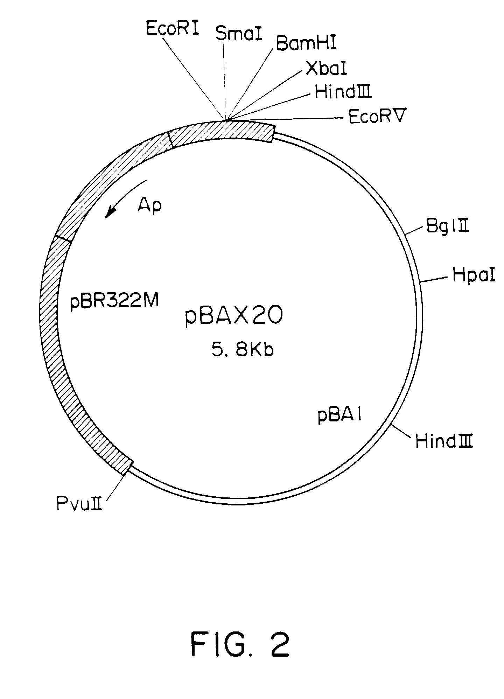

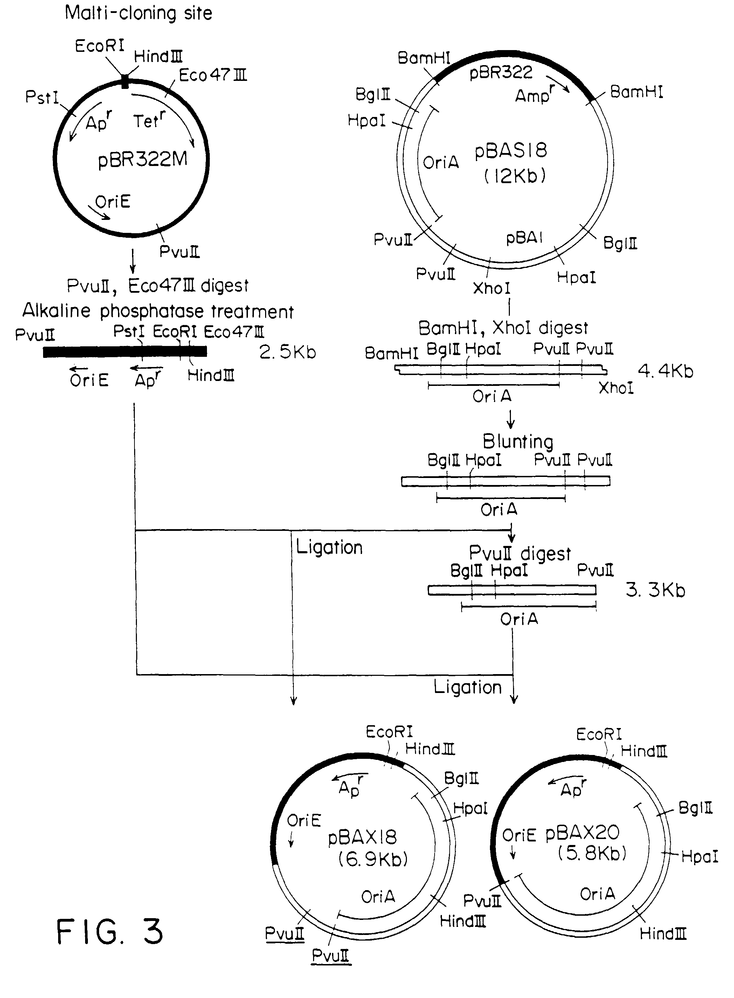

Fig. 1 is a restriction endonuclease map of plasmid pBAX 18, Fig. 2 is a restriction endonuclease map of plasmid pBAX 20, and Fig. 3 is a construction drawing of plasmids pBAX 18 and pBAX 20.

The numbers of recognition sites of plasmids pBAX 18 and pBAX 20 with various restriction endonucleases and the lengthes (kb) of digested fragments thereof with the restriction endonucleases are as shown in the following Table 1. Further, the restriction endonuclease maps of plasmids pBAX 18 and pBAX 20 are shown in Figs. 1 to 2.

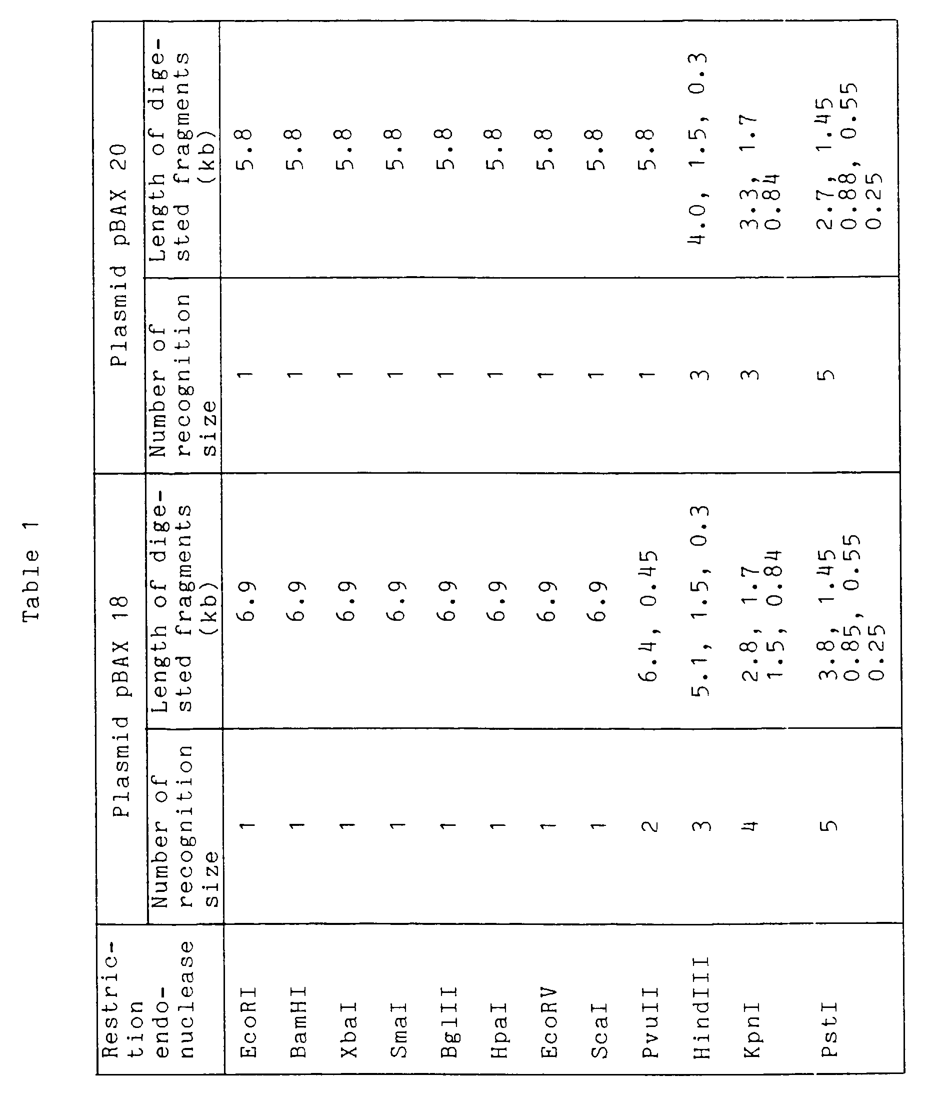

The plasmids pBAX 18 and pBAX 20 of this invention having the above-mentioned characteristics can, for example, be prepared as follows.

First, the OriA-containing DNA fragment of plasmid pBA 1 derived from Anacystic nidulans can be prepared, for example, by cloning a shuttle vector pBAS 18 in Anacystic nidulans and Escherichia coli [refer to K. Shinozaki et al., Gene 19:221-224 (1982)] in Escherichia coli according to a conventional method (T. Maniatis et al., Molecular Cloning-a Laboratory Manual-, published by Cold Spring Hobor Laboratory), and then cutting DNA fragments out from the cloned plasmid pBAS 18 according to a coventional method (T. Maniatis et al., Molecular Cloning) and using the combination of restriction endonuclease BamHI and XhoI or the combination of restriction endonucleases BamHI and PvuII. Thereby, OriA-containing DNA fragments are obtained having sizes of about 4.1 kb and about 3.3 kb respectively. The 5' projecting and of these DNA fragments can be blunted by T₄ DNA polymerase, if necessary.

On the other hand, the MC region of plasmid pUC can, for example, be prepared by cutting it out from plasmid pUC 18 with restriction endonucleases EcoRI and Hind III.

Further, it is convenient to use plasmid pBR 322, a typical Col E1 plasmid as a source of the DNA fragment which contains the OriE region of Col E1 plasmid and wherein the gene defined by the rop region is removed.

The thus prepared MC region is combined with the restriction endonucleases EcoRI and Hind III-treated plasmid pBR 322, and T₄ DNA ligase is made to act thereon to prepare plasmid pBR 322M wherein the above MC region is integrated into plasmid pBR 322. Then, a DNA fragment of about 2.5 kb which is obtained by treating this plasmid pBR 322 M with restriction endonuclease Eco 47III and PvuII and contains both OriE and MC region is combined with the OriA-containing DNA fragment prepared as above, and T₄ DNA ligase is made to act thereon to obtain the desired plasmids pBAX 18 and pBAX 20.

The construction drawing of plasmids pBAX 18 and pBAX 20 are shown in Fig. 3 and more specific preparation methods thereon are in more detail described later in examples.

Thus prepared plasmids of this invention are shuttle vectors having the OriE region making its autonomous replication in the cells of Escherichia coli possible and the OriA region making its autonomous replication in the cells of Anacystis nidulans possible. Therefore, the plasmids of the invention can be prepared in a large amount using Escherichia coli proliferating in a high speed, and by using the plasmid DNA thus prepared in a large amount it is possible to efficiently transform cells of cyanobacteria such as Anacystis nidulans.

Further, Since the multicloning region is introduced in the plasmid of the invention, as recognition sites for inserting (foreign) genes recognition sites such as SmaI, BamHI, XbaI, ScaI and EcoRV are utilizable in addition to the EcoRI recognition site solely used in plasmid pBAS 18.

Further, the plasmid of the invention can be miniaturized up to about a half in size (6.9 kb to 5.8 kb) of plasmid pBAS 18 (12 kb) because the DNA parts other than the OriA region of plasmid pBA1 is removed in the present plasmid. Consequently, it is possible to introduce into the plasmid of the invention a (foreign) gene larger than a (foreign) gene which can be introduced into plasmid pBAS 18.

Introduction of the plasmid of this invention having the above characteristics on gene manipulation into a host cell can be carried out by a method known per se, for example by a method disclosed in literatures such as D. A. Lightfoot et al., J. General Microbial 134: 1509-1514 (1988).

The transformation frequency of the thus transformed Anacystis nidulans 6301 can be about 1,000-fold, compared with the value when the strain was transformed with plasmid pBAS 18. Therefore, by using the plasmid of the invention it is possible, even when a (foreign gene) is inserted, to easily select cells having the plasmid wherein the desired gene is inserted.

Mentioned as structural genes capable of being integrated into the plasmid of the invention are structural genes derived from Anacystis nidulans, structural genes derived from other cyanobacteria cells, structural genes derived from other bacteria, structural genes derived from higher animals and plants, etc., and thus therein origins are not limited at all. Further, chemically synthesized structural genes also be utilized as structural genes capable of being integrated into the plasmid of the invention. Examples of such genes are chemically synthesized human superoxide dismutases [h-SOD, h-SOD-Ala⁶ (refer to Japanese Laid-Open Patent Publication No. 156884/1990), etc.].

The plasmid of this invention is more specifically described below according to examples.

### Example 1

### (1) Introduction of a multicloning site (derived from pUC 18) into pBR 322

### A) EcoRI-Hind III digestion and alkaline phosphatase treatment of pBR 322

To 20 µl (10 µg) of a pBR 322 DNA solution were added 40 µl of a 5 x Hind III buffer (50 mM Tris-HCl (pH 7.5), 35 mM MgCl₂, 300 mM NaCl), 80 units (10 µl) of Hind III (produced by Takara Shuzo Co., Ltd.) and 130 µl of sterilized water, and the mixture was subjected to incubation at 37°C for 2 hours. After the reaction, to the resulting solution were added to 40 µl of a 5 x EcoRI buffer (500 mM Tris-HCl (pH 7.5), 35 mM MgCl₂, 250 mM NaCl, 35 mM 2-mercaptoethanol, 0.05 % bovine serum albumin), 120 units (10 µl) of EcoRI (produced by Takara Shuzo Co., Ltd.) and 50 µl of sterilized water, and the mixture was subjected to reaction at 37°C for 2 hours. After the reaction, the mixture was subjected to phenol-chloroform treatment and ethanol precipitation, and the resulting DNA is collected and dissolved in 100 µl of 0.1 M Tris-HCl (pH 8.0). To this solution was added 10 µl of an alkaline phosphatase (produced by Takara Shuzo Co., Ltd.) solution (1 unit/10 µl alkaline phosphatase, 10 mM Tris-HCl (pH 7.5), 50 mM NaCl, 1 mM ZnSO₄), and the mixture was subjected to incubation at 37°C for 1 hour. After the reaction, 10 µl of the alkaline phosphatase solution was further added and the mixture was further subjected to incubation at 65°C for 30 minutes. The resulting solution was treated with phenol-chloroform and then subjected to ethanol precipitation, and the resulting DNA was recovered.

### B) Isolation of a multicloning site (EcoRI-Hind III) from pUC 18

Two Eppendorf tubes were prepared in each of which to 30 µl (20 µg) of a pUC 18 DNA solution had been added 20 µl of a 10 x K buffer (200 mM Tris-HCl (pH 8.5), 100 mM MgCl₂, 10 mM DTT, 100 mM KCl), 80 units (10 µl) of Hind III and 140 µl of sterilized water and which contained the mixture, and each mixture therein was subjected to incubation at 37°C for 3 hours After the reaction, the mixture was treated to phenol-chloroform and subjected to ethanol precipitation, and the resulting DNA was collected and dissolved in 112.5 µl of sterilized water. To each of the resulting solutions were added 30 µl of a 5 x EcoRI buffer and 190 units (7.5 µl) of EcoRI, and the mixture was subjected to incubation at 37°c for 3 hours. DNAs were precipitation with ethanol and recovered and subjected to 1.5 % agarose gel electrophoresis to separate the desired DNA fragment (about 50 bp). This DNA was electrically eluted from the gel, treated with phenol-chloroform and purified by ethanol precipitation.

### C) Ligation of pBR 322 (degested with EcoRI-Hind III) to the multicloning

To 0.2 µg (1 µl) of pBR 322 (EcoRI-Hind III, AP treatment) DNA and 0.2 µg (1 µl) of the multicloning site DNA were added 1 µl of TE (10 mM Tris-HCl (pH 8.0), 1 mM EDTA) and 24 µl of the Takara ligation kit A solution, and the mixture was sufficiently stirred. To this solution was added 3 µl of the Takara ligation kit B solution and the mixture was subjected to incubation at 16°C for 4 hours.

### D) Cloning of plasmid pBR 322M

To 3 µl of (40 ng) of the resulting ligation solution was added 200 µl of a cell suspension of E. coli HB 101 treated with 50 mM CaCl₂, and the mixture was gently mixed. This mixture was subjected to incubation in ice water for 30 minutes and then to incubation at 42°C for 2 minutes to make the cells take DNAs therein. To this suspension was added 1.8 ml of a 2YT (16 g/l trypton, 10 g/l yeast extract, 5 g/l NaCl) liquid medium, and the mixture was subjected to shaking culture at 37°C for 1 hour and then plated on a LB (10 g/l trypton, 8 g/l NaCl, 5 g/l yeast extract) agar medium (containing 50 µg/ml ampicillin). Plasmids were prepared from the resulting colonies and their restriction endonuclease maps were analyzed to screen colonies having the desired plasmid (pBR 322M). The screened colonies were cultured in 200 ml of a 2YT liquid medium (containing 100 µg/ml ampicillin) and the plasmid DNA was prepared in a large amount according to the SDS-alkali method.

### (2) Isolation of PvuII-Eco 47III fragment (2550 bp)

Three Eppendorf tubes were prepared in each of which to 10 µg (10 µl) of pBR 322M plasmid DNA prepared in the above (1) had been added 20 µl of a 10 x M buffer (100 mM Tris-HCl (pH 7.5), 100 mM MgCl₂, 10 mM DTT, 500 mM NaCl), 120 units (10 µl) of PvuII (produced by Takara Shuzo Co., Ltd.) and sterilized water to make the total volume of 200 µl and which contained the mixture. These tubes were subjected to incubation at 37°C for 3 hours. After the reaction, the mixtures were treated with phenol-chloroform, and DNAs were collected by ethanol precipitation and dissolved in 174 µl portions of sterilized water respectively. To these solutions were added 20 µl portions of a 10 x H buffer and 24 units portions of Eco47 III (produced by Takara Shuzo Co., Ltd.), and the mixtures were incubated at 37°C for 3 hours respectively. DNAs were recovered by ethanol precipation and the desired DNA fragment (2550 bp) was separated by 1.5 % agarose gel electroporesis. The separated DNA fragment was purified using Geneclean and formed into 50 µl of a 0.1 M Tris-HCl (pH 8.0) solution. To this solution was added 5 µl of an alkaline phosphates solution and the mixture was subjected to incubation at 37°C for 1 hour. After the reaction, 5 µl of the alkaline phosphatase solution and the mixture was further subjected to incubation at 65°C for 30 minutes. After the reaction, the mixture was subjected to phenol-chloroform treatment and subsequent ethanol precipitation, and the resulting DNAs were collected and dissolved in 20 µl of TE.

### (3) Separation of the replication initiation point in A. nidulans of pBAS 18

Into E. coli HB 101 was introduced the shuttle vector pBAS 18 (K. Shinozaki et al., Gene 19:221-224 (1982)) between Escherichia coli and A. nidulans, wherein an endogenous plasmid (pBA 1, digested with BamHI) of A. nidulans 6301 strain had been inserted into the BamHI site of pBR322. The resulting strain was cultured in an LB liquid medium (containing 50 µg/ml ampicillin) and the vector plasmid was prepared in a large amount using the SDS-alkali method. To 14 µg (20 µl) of the prepared pBAS 18 DNA were added 20 µl of a 10 x K buffer, 100 units (10 µl) of BamHI (produced by Takara Shuzo Co., Ltd.) and sterilized water to make the total volume of 200 µl in an Eppendorf tube. Three Eppendorf tubes were prepared containing the same contents as above, and the contents were subjected to incubation at 30°C for 3 hours. After the reaction, DNAs were recovered by ethanol precipitation, and the desired DNA fragment (pBA 1, about 8.0 kbp) was separated by 1 % agarose gel electrophoresis and purified by Geneclean. To 2 µg (5 µl) of the separated and purified pBA 1 (digested with BamHI) DNA were added 5 µl of a 10 x K buffer, 24 units (2 µl) of XhoI (produced by Takara Shuzo Co., Ltd.) and 38 µl of sterilized water, and the mixture was subjected to incubation at 37°C for 3 hours. After the reaction, the mixture was treated with phenol-chloroform and the DNA was collected by ethanol precipitation. The both ends of the obtained DNA which is a digest of pBA1 with BamHI-XhoI were blunted using the Takara Blanting kit.

### (4) Construction of a miniaturized E. coli-A. nidulans shuttle vector pBAX 18 (6.9 kbp)

To 40 ng (2 µl) of the blunted DNA and 200 ng (4 µl) of the PuvII-Eco47III fragment DNA was added 48 µl of the Takara ligation kit A solution, followed by sufficient stirring. 6µl of the B solution was added and incubation was carried out at 16°C for 4 hours. The E. coli HB 101 strain was transformed using this solution and then plated on an LB agar medium (containing 50 µg/ml ampicillin and 1.5 % agar) to obtain colonies. Plasmids were prepared from the obtained colonies and analyzed for restriction endonuclease map to screen colonies carrying the desired plasmid pBAX 18. The screened colonies were cultured in 45 ml portions of a 2YT liquid medium (containing 100 µg/ml ampicillin) and the plasmid DNA was prepared by the SDS-alkali method.

### (5) Preparation of pBAX 20 (about 5.8 kb)

To 1 µg (u µl) of BamHI-XhoI (blunted) DNA fragment prepared in (3) were added 2 µl of a 10 x M buffer, 1 µl (12 units) of PvuII and 13 µl of sterilized water, and incubation was carried out at 37°C for 3 hours. To 2 µl (100 ng) of this reaction solution were added 100 ng (2 µl) of the PvuII-Eco 47 DNA fragment prepared in (2) and 16 µl of Takara ligation Kit A solution, and after sufficient stirring the B solution was added and then incubation was carried out at 16°c for 2 hours. After the reaction, E. coli HB 101 strain was transformed using this solution and plated on an LB agar medium (containing 50 µg/ml ampicillin and 1.5 % agar) to obtain colonies. Colonies carrying the desired plasmid (pBAX 20) were screened from the obtained colonies and cultured in 200 ml of a 2YT liquid medium (containing 100 µg/ml ampicillin) and the plasmid DNA was prepared by the SDS-alkali method.

### Example 2

### Transformation of A. nidulans 6301

The cells cultured in 100 ml of a BG-11 liquid medium for 1 to 5 days were collected by centrifugation at 8,000 rpm for 5 minutes and suspended in 10 ml of a fresh liquid medium 10⁸ to 10⁹ cells/ml). One ml portions of this cell suspension were poured into polyethylene tubes (Falcon 2059), and the plasmid DNAs prepared in Example 1 were added in the respective tubes in a concentration of 1 µg (pBAS 18 is 10 µg). These tubes were covered with aluminum foils respectively, culture was carried out at 30°c overnight, the aluminum foils were removed, and culture was further continued at 30°C under irradiation with light (1,000-2,000 luxes). These cell suspensions were diluted 5-fold (those to which pBAS 18 had been added were not diluted) and plated in 100 to 500 µl proportions thereof on a BG-11 agar medium (containing 1 µg/ml ampicillin and 1 mM sodium thiosulfate and 1.5 % agar). These plates were culture subjected to culture under irradiation with light (2,000-3,000 luxes) for 4 to 10 days.

The transformation frequency of the thus obtained colonies were 10⁻³ to 10⁻⁴ cells/µg plasmid DNA in case where the plasmids of the invention were used, and thus about 1,000-fold frequencies were obtained, compared to the case (10⁻⁷ cells/µg plasmid DNA) where plasmid pBAS 18 was used.

### Industrial applicability

The plasmid of this invention can be prepared in a large amount using Escherichia coli and replication in the cells of both Escherichia coli and cyanobacteria, and are useful as a shuttle vector for expression of various structural genes in the cells of cyanobacteria.

## Claims (Claims for the following Contracting State(s): AT, BE, CH, LI, DE, DK, FR, GB, IT, NL, SE)

1. A shuttle vector plasmid replicable in the cells of Escherichia coli and cyanobacteria, which contains
(a) the OriA region of plasmid pBA1 derived from Anacystis nidulans,
(b) a multicloning region comprising a sequence having various restriction endonuclease recognition sites and
(c) the region of colicin E1 plasmid, which contains the OriE region thereof and wherein the gene defined by the rop functional region thereof is removed.

2. The plasmid of claim 1 wherein said OriA region (a) is an OriA region-containing DNA fragment whose size is 2.8 to 8.0 kb when it is cut out from plasmid pBAl.

3. The plasmid of claim 1 wherein said OriE region (c) is a DNA fragment which contains the OriE region and wherein the gene defined by the rop region is removed, the size of the DNA fragment being 1.5 to 4.1 kb when it is cut out from plasmid pBR 322.

4. The plasmid of any of claims 1 to 3 wherein said OriE-containing region further contains an ampicillin-resistance gene (Amp^{r}).

5. A vector plasmid according to claim 1 having a size of about 6.9 kb and characterized by the restriction endonuclease map shown in Fig. 1.

6. A vector plasmid according to claim 1 having a size of about 5.8 kb and characterized by the restriction endonuclease map shown in Fig. 2.

7. A cyanobacterium transformed with the plasmid of claim 1 further containing a structural gene.

8. A cyanobacterium of claim 7 which is Anacystis nidulans 6301 strain.

## Claims (Claims for the following Contracting State(s): ES)

1. A process for producing a shuttle vector plasmid replicable in the cells of Escherichia coli and cyanobacteria, comprising combining:
(a) the OriA region of plasmid pBA1 derived from Anacystis nidulans, with
(b) a multicloning region comprising a sequence having various restriction endonuclease recognition sites and
(c) the region of colicin E1 plasmid, which contains the OriE region thereof and wherein the gene defined by the rop functional region thereof is removed.

2. The process of claim 1 wherein said OriA region (a) is an OriA region-containing DNA fragment whose size is 2.8 to 8.0 kb when it is cut out from plasmid pBA1.

3. The process of claim 1 wherein said OriE region (c) is a DNA fragment which contains the OriE region and wherein the gene defined by the rop region is removed, the size of the DNA fragment being 1.5 to 4.1 kb when it is cut out from plasmid pBR 322.

4. The process of any of claims 1 to 3 wherein said OriE-containing region further contains an ampicillin-resistance gene (Amp^{r}).

5. A process according to claim 1 wherein the vector plasmid has a size of about 6.9 kb and is characterized by the restriction endonuclease map shown in Fig. 1.

6. A process according to claim 1 wherein the vector plasmid has a size of about 5.8 kb and is characterized by the restriction endonuclease map shown in Fig. 2.

7. A process for introducing a structural gene into cyanobacterium comprising transforming cyanobacterium with a plasmid vector containing a structural gene, the plasmid vector being produced by the process of any of claims 1 to 6.

8. The process of claim 7 wherein the cyanobacterium is Anacystis nidulans 6301 strain.

9. A shuttle vector plasmid replicable in the cells of Escherichia coli and cyanobacteria, which contains
(a) the OriA region of plasmid pBA1 derived from Anacystis nidulans,
(b) a multicloning region comprising a sequence having various restriction endonuclease recognition sites and
(c) the region of colicin E1 plasmid, which contains the OriE region thereof and wherein the gene defined by the rop functional region thereof is removed.

10. The plasmid of claim 9 wherein said OriA region (a) is a OriA region-containing DNA fragment whose size is 2.8 to 8.0 kb when it is cut out from plasmid pBA1.

11. The plasmid of claim 9 wherein said OriE region (c) is an DNA fragment which contains the OriE region and wherein the gene defined by the rop region is removed, the size of the DNA fragment being 1.5 to 4.1 kb when it is cut out from plasmid pBR 322.

12. The plasmid or any of claims 9 to 11 wherein said OriE-containing region further contains an ampicillin-resistance gene (Amp^{r}).

13. A vector plasmid according to claim 9 having a size of about 6.9 kb and characterized by the restriction endonuclease map shown in Fig. 1.

14. A vector plasmid according to claim 9 having a size of about 5.8 kb and characterized by the restriction endonuclease map shown in Fig. 2.

15. A cyanobacterium transformed with the plasmid of claim 9 further containing a structural gene.

16. A cyanobacterium of claim 15 which is Anacystis nidulans 6301 strain.

## Patentansprüche (Patentansprüche für folgende(n) Vertragsstaat(en): AT, BE, CH, LI, DE, DK, FR, GB, IT, NL, SE)

1. Shuttlevektor-Plasmid, das in den Zellen von Escherichia coli und Cyanobakterien replikationsfähig ist, enthaltend
(a) die OriA-Region des Plasmids pBA1, abgeleitet aus Anacystis nidulans,
(b) eine Mehrfachklonierungsregion, welche eine Sequenz umfaßt, die verschiedene Restriktionsendonuklease-Erkennungsstellen besitzt, und
(c) die Region des Colicin E1-Plasmides, welche die OriE-Region davon enthält und worin das durch die funktionelle rop-Region definierte Gen daraus entfernt ist.

2. Plasmid von Anspruch 1, wobei die OriA-Region (a) ein die OriA-Region enthaltendes DNA-Fragment ist, dessen Größe sich auf 2,8 bis 8,0 kb beläuft, wenn es aus dem Plasmid pBA1 herausgeschnitten wird.

3. Plasmid von Anspruch 1, wobei die OriE-Region (c) ein DNA-Fragment ist, das die OriE-Region enthält und worin das durch die rop-Region definierte Gen entfernt ist, wobei die Größe des DNA-Fragmentes 1,5 bis 4,1 kb beträgt, wenn es aus dem Plasmid pBR322 herausgeschnitten wird.

4. Plasmid von mindestens einem der Ansprüche 1 bis 3, wobei die OriE-enthaltende Region ferner ein Ampicillin-Resistenzgen (Amp^{r}) enthält.

5. Vektorplasmid gemäß Anspruch 1, das eine Größe von etwa 6,9 kb aufweist und durch die in Fig. 1 gezeigte Restriktionsendonuklease-Karte gekennzeichnet ist.

6. Vektorplasmid gemäß Anspruch 1, das eine Größe von etwa 5,8 kb aufweist und durch die in Fig. 2 gezeigte Restriktionsendonuklease-Karte gekennzeichnet ist.

7. Cyanobakterium, transformiert mit dem Plasmid von Anspruch 1, das ferner ein Strukturgen enthält.

8. Cyanobakterium von Anspruch 7, bei dem es sich um den Anacystis nidulans-Stamm 6301 handelt.

## Patentansprüche (Patentansprüche für folgende(n) Vertragsstaat(en): ES)

1. Verfahren zur Herstellung eines Shuttlevektor-Plasmids, das in den Zellen von Escherichia coli und Cyanobakterien replikationsfähig ist, umfassend das Kombinieren:
(a) der OriA-Region des aus Anacystis nidulans abgeleiteten Plasmids pBA1 mit
(b) einer Mehrfachklonierungsregion, welche eine Sequenz umfaßt, die verschiedene Restriktionsendonuklease-Erkennungsstellen besitzt, und
(c) der Region des Colicin E1-Plasmides, welche die OriE-Region davon enthält und worin das durch die funktionelle rop-Region definierte Gen daraus entfernt ist.

2. Verfahren von Anspruch 1, wobei die OriA-Region (a) ein die OriA-Region enthaltendes DNA-Fragment ist, dessen Größe sich auf 2,8 bis 8,0 kb beläuft, wenn es aus dem Plasmid pBAl herausgeschnitten wird.

3. Verfahren von Anspruch 1, wobei die OriE-Region (c) ein DNA-Fragment ist, das die OriE-Region enthält und worin das durch die rop-Region definierte Gen entfernt ist, wobei die Größe des DNA-Fragmentes 1,5 bis 4,1 kb beträgt, wenn es aus dem Plasmid pBR322 herausgeschnitten wird.

4. Verfahren von mindestens einem der Ansprüche 1 bis 3, wobei die OriE-enthaltende Region ferner ein Ampicillin-Resistenzgen (Amp^{r}) enthält.

5. Verfahren gemäß Anspruch 1, wobei das Vektorplasmid eine Größe von etwa 6,9 kb aufweist und durch die in Fig. 1 gezeigte Restriktionsendonuklease-Karte gekennzeichnet ist.

6. Verfahren gemäß Anspruch 1, wobei das Vektorplasmid eine Größe von etwa 5,8 kb aufweist und durch die in Fig. 2 gezeigte Restriktionsendonuklease-Karte gekennzeichnet ist.

7. Verfahren zur Einführung eines Strukturgens in ein Cyanobakterium, umfassend das Transformieren eines Cyanobakteriums mit einem ein Strukturgen enthaltenden Plasmidvektor, wobei der Plasmidvektor durch das Verfahren von mindestens einem der Ansprüche 1 bis 6 hergestellt wird.

8. Verfahren von Anspruch 7, wobei es sich bei dem Cyanobakterium um den Anacystis nidulans-Stamm 6301 handelt.

9. Shuttlevektor-Plasmid, das in den Zellen von Escherichia coli und Cyanobakterien replikationsfähig ist, enthaltend
(a) die OriA-Region des Plasmids pBA1, abgeleitet aus Anacystis nidulans,
(b) eine Mehrfachklonierungsregion, welche eine Sequenz umfaßt, die verschiedene Restriktionsendonuklease-Erkennungsstellen besitzt, und
(c) die Region des Colicin E1-Plasmides, welche die OriE-Region davon enthält und worin das durch die funktionelle rop-Region definierte Gen daraus entfernt ist.

10. Plasmid von Anspruch 9, wobei die OriA-Region (a) ein die OriA-Region enthaltendes DNA-Fragment ist, dessen Größe sich auf 2,8 bis 8,0 kb beläuft, wenn es aus dem Plasmid pBAl herausgeschnitten wird.

11. Plasmid von Anspruch 9, wobei die OriE-Region (c) ein DNA-Fragment ist, das die OriE-Region enthält und worin das durch die rop-Region definierte Gen entfernt ist, wobei die Größe des DNA-Fragmentes 1,5 bis 4,1 kb beträgt, wenn es aus dem Plasmid pBR322 herausgeschnitten wird.

12. Plasmid von mindestens einem der Ansprüche 9 bis 11, wobei die OriE-enthaltende Region ferner ein Ampicillin-Resistenzgen (Amp^{r}) enthält.

13. Vektorplasmid gemäß Anspruch 9, das eine Größe von etwa 6,9 kb aufweist und durch die in Fig. 1 gezeigte Restriktionsendonuklease-Karte gekennzeichnet ist.

14. Vektorplasmid gemäß Anspruch 9, das eine Größe von etwa 5,8 kb aufweist und durch die in Fig. 2 gezeigte Restriktionsendonuklease-Karte gekennzeichnet ist.

15. Cyanobakterium, transformiert mit dem Plasmid von Anspruch 9, das ferner ein Strukturgen enthält.

16. Cyanobakterium von Anspruch 15, bei dem es sich um den Anacystis nidulans-Stamm 6301 handelt.

## Revendications (Revendications pour l'(les) Etat(s) contractant(s) suivant(s): AT, BE, CH, LI, DE, DK, FR, GB, IT, NL, SE)

1. Plasmide-vecteur navette réplicable dans les cellules d*'Escherichia coli* et de cyanobactéries, contenant
(a) la région OriA du plasmide pBA1 dérivé d*'Anacystis nidulans,*
(b) une région de multiclonage comprenant une séquence ayant divers sites de reconnaissance d'endonucléases de restriction et
(c) la région du plasmide El de colicine, qui contient la région OriE de celui-ci et dont le gène défini par la région fonctionnelle rop de celui-ci est enlevé.

2. Plasmide selon la revendication 1, dans lequel ladite région OriA (a) est un fragment d'ADN contenant la région OriA, dont la dimension est de 2,8 à 8,0 kb quand il est coupé du plasmide pBA1.

3. Plasmide selon la revendication 1, dans lequel ladite région OriE (c) est un fragment d'ADN qui contient la région OriE et dont le gène défini par la région rop est enlevé, la dimension du fragment d'ADN étant de 1,5 à 4,1 kb quand il est coupé du plasmide pBR 322.

4. Plasmide selon l'une quelconque des revendications 1 à 3, dans lequel ladite région contenant OriE contient, en outre, un gène de résistance à l'ampicilline (Amp^{r}).

5. Plasmide-vecteur selon la revendication 1, ayant une dimension d'environ 6,9 kb et caractérisé par la carte des endonucléases de restriction représentée sur la figure 1.

6. Plasmide-vecteur selon la revendication 1, ayant une dimension d'environ 5,8 kb et caractérisé par la carte des endonucléases de restriction représentée sur la figure 2.

7. Cyanobactérie transformée avec le plasmide selon la revendication 1, contenant, en outre, un gène de structure.

8. Cyanobactérie selon la revendication 7, qui est la souche 6301 d'*Anacystis nidulans.*

## Revendications (Revendications pour l'(les) Etat(s) contractant(s) suivant(s): ES)

1. Procédé de production d'un plasmide-vecteur navette réplicable dans les cellules d*'Escherichia coli* et de cyanobactéries, comprenant la combinaison de :
(a) la région OriA du plasmide pBA1 dérivé d*'Anacystis nidulans* avec
(b) une région de multiclonage comprenant une séquence ayant divers sites de reconnaissance d'endonucléases de restriction et
(c) la région du plasmide E1 de colicine, qui contient la région OriE de celui-ci et dont le gène défini par la région fonctionnelle rop de celui-ci est enlevé.

2. Procédé selon la revendication 1, dans lequel ladite région OriA (a) est un fragment d'ADN contenant la région OriA, dont la dimension est de 2,8 à 8,0 kb quand il est coupé du plasmide pBA1.

3. Procédé selon la revendication 1, dans lequel ladite région OriE (c) est un fragment d'ADN qui contient la région OriE et dont le gène défini par la région rop est enlevé, la dimension du fragment d'ADN étant de 1,5 à 4,1 kb quand il est coupé du plasmide pBR 322.

4. Procédé selon l'une quelconque des revendications 1 à 3, dans lequel ladite région contenant OriE contient, en outre, un gène de résistance à l'ampicilline (Amp^{r}).

5. Procédé selon la revendication 1, dans lequel le plasmide-vecteur a une dimension d'environ 6,9 kb et est caractérisé par la carte des endonucléases de restriction représentée sur la figure 1.

6. Procédé selon la revendication 1, dans lequel le plasmide-vecteur a une dimension d'environ 5,8 kb et est caractérisé par la carte des endonucléases de restriction représentée sur la figure 2.

7. Procédé pour introduire un gène de structure dans une cyanobactérie, comprenant la transformation de la cyanobactérie avec un plasmide-vecteur contenant un gène de structure, le plasmide-vecteur étant produit au moyen du procédé selon l'une quelconque des revendications 1 à 6.

8. Procédé selon la revendication 7, dans lequel la cyanobactérie est la souche 6301 d*'Anacystis nidulans.*

9. Plasmide-vecteur navette réplicable dans les cellules d*'Escherichia coli* et de cyanobactéries, contenant
(a) la région OriA du plasmide pBA1 dérivé d*'Anacystis nidulans,*
(b) une région de multiclonage comprenant une séquence ayant divers sites de reconnaissance d'endonucléases de restriction et
(c) la région du plasmide El de colicine, qui contient la région OriE de celui-ci et dont le gène défini par la région fonctionnelle rop de celui-ci est enlevé.

10. Plasmide selon la revendication 9, dans lequel ladite région OriA (a) est un fragment d'ADN contenant la région OriA, dont la dimension est de 2,8 à 8,0 kb quand il est coupé du plasmide pBA1.

11. Plasmide selon la revendication 9, dans lequel ladite région OriE (c) est un fragment d'ADN qui contient la région OriE et dont le gène défini par la région rop est enlevé, la dimension du fragment d'ADN étant de 1,5 à 4,1 kb quand il est coupé du plasmide pBR 322.

12. Plasmide selon l'une quelconque des revendications 9 à 11, dans lequel ladite région contenant OriE contient, en outre, un gène de résistance à l'ampicilline (Amp^{r}).

13. Plasmide-vecteur selon la revendication 9, ayant une dimension d'environ 6,9 kb et caractérisé par la carte des endonucléases de restriction représentée sur la figure 1.

14. Plasmide-vecteur selon la revendication 9, ayant une dimension d'environ 5,8 kb et caractérisé par la carte des endonucléases de restriction représentée sur la figure 2.

15. Cyanobactérie transformée avec le plasmide selon la revendication 9, contenant, en outre, un gène de structure.

16. Cyanobactérie selon la revendication 15, qui est la souche 6301 d*'Anacystis nidulans.*
